# EUROPEAN PATENT SPECIFICATION

(11) **EP 3 675 757 B1**
(45) Date of publication and mention of the grant of the patent: **21.04.2021**
(21) Application number: 18758849.6
(22) Date of filing: 15.08.2018
(51) Int. Cl.: A61B 18/14, A61B 8/12, A61B 90/00, A61B 18/00

(54) **ABLATION CATHETER, CATHETER ARRANGEMENT AND SYSTEM FOR PROVIDING ABLATIVE TREATMENT**
ABLATIONSKATHETER, KATHETERANORDNUNG UND SYSTEM ZUR BEREITSTELLUNG EINER ABLATIVEN BEHANDLUNG
CATHÉTER D'ABLATION, AGENCEMENT DE CATHÉTER ET SYSTÈME POUR FOURNIR UN TRAITEMENT ABLATIF

(30) Priority: 29.08.2017 EP 17188237
(43) Date of publication of application: 08.07.2020
(73) Proprietor: Koninklijke Philips N.V., 5656 AG Eindhoven (NL)
(72) Inventor: GIJSBERS, Gerardus, Henricus, Maria, 5656 AE Eindhoven (NL); KOLEN, Alexander, Franciscus, 5656 AE Eindhoven (NL); BELT, Harm, Jan, Willem, 5656 AE Eindhoven (NL); HARKS, Godefridus, Antonius, 5656 AE Eindhoven (NL)
(74) Representative: Philips Intellectual Property & Standards
(86) International application number: PCT/EP2018/072080
(87) International publication number: WO 2019/042769

(56) References cited:
- WO-A1-2015/148470
- US-A1- 2010 204 561
- US-A1- 2014 121 470
- US-A1- 2016 051 321

## Description

### FIELD OF THE INVENTION

The present invention relates to an ablation catheter, and a catheter arrangement comprising the same. The present invention further relates to a system for providing ablative treatment, the system including the ablation catheter or catheter arrangement.

### BACKGROUND OF THE INVENTION

Ventricular tachycardia (VT) is a life-threatening arrhythmia that is common to all forms of heart disease and a cause of sudden death. Arrhythmia may be caused by the presence of an additional electrical pathway in the heart, which may be due to a scar from an infarction or replacement fibrosis.

Ablative treatment may be administered to patients suffering from cardiac arrhythmia, such as VT. Ablation of electrically active cardiac tissue generates electrically inactive scar tissue. This scar tissue blocks the additional electrical pathway thereby alleviating the arrhythmia.

Several different ablation methods have been developed. One particular example is radiofrequency (RF) ablation in which RF energy is supplied to the tissue which is targeted for ablation. RF ablation causes resistive heating in the tissue, leading to tissue coagulation and thus permanent tissue damage.

However, the therapeutic success of this technique is limited as in many patients the scar tissue which is causing the arrhythmia may lie in the mid-myocardial region and it is difficult if not impossible to assess whether the target myocardial tissue between the endocardial surface where RF ablation energy is applied and the deeper scar region is coagulated. This has resulted in a disappointing acute success rate of between 49-89%; long-term recurrences occur in 34-37% of cases (Tanawuttiwat T et al., European Heart Journal, 2016, vol. 37, pages 594-609).

Document WO/2015/148470 discloses a method of generating a graphical representation of cardiac information on a display screen. The method comprises: electronically creating or acquiring an anatomical model of the heart including multiple cardiac locations; electronically determining a data set of source information corresponding to cardiac activity at the multiple cardiac locations; electronically rendering the data set of source information in relation to the multiple cardiac locations on the display screen. Systems and devices for providing a graphical representation of cardiac information are also provided.

It thus remains a challenge to improve the assessment of scar position and depth, and to follow the progress of the ablation treatment as it is being administered so as to increase the effectiveness of ablation treatment, e.g. for conditions such as VT, e.g. ischemic VT, as well as for other cardiac pathologies such as atrial fibrillation.

### SUMMARY OF THE INVENTION

The present invention seeks to address at least some of the abovementioned problems.

The invention is defined by the claims.

According to an aspect of the present invention there is provided an ablation catheter for ablative treatment of ventricular tachycardia comprising: a shaft; an ablation element mounted on the shaft; a plurality of ultrasound transducer arrays for obtaining images of myocardial tissue, the arrays being spaced relative to each other around the shaft, wherein the catheter is configurable to adopt a folded configuration in which the arrays extend along the shaft, and a deployed configuration in which the arrays are more separated from the shaft than in the folded configuration.

The separation of the ultrasound transducer arrays from the shaft in the deployed configuration may result in at least one of the ultrasound transducer arrays projecting towards the target tissue, i.e. the tissue to be ablated. Projection of an ultrasound transducer array towards, e.g. against, the target tissue in this manner may facilitate assessment of the position of a scar lesion, e.g. from an infarction or replacement fibrosis, residing in the target tissue. Ultrasound imaging using the deployed ultrasound transducer arrays may thus enable effective guiding of the ablation treatment delivered via the ablation element to the target tissue.

Furthermore, such projection of at least one of the deployed ultrasound transducer arrays towards the target tissue may assist in monitoring the ablation process. The capability to follow the ablation process using the deployed ultrasound transducer arrays may therefore assist to ensure that the ablation treatment is carried out to the requisite extent, and is terminated when sufficient tissue ablation, e.g. coagulation, is deemed to have occurred.

Insertion of the ablation catheter into a patient in the deployed configuration risks causing unintended harm to the patient and damage to the ultrasound transducer arrays. For this reason, the ablation catheter is capable of adopting a folded configuration in which the arrays extend along the shaft of the catheter, i.e. against or close to the shaft, thereby enabling insertion and guiding of the ablation catheter with less risk to the patient, and with less risk of damaging the ultrasound transducer arrays.

The ultrasound transducer arrays may extend outwardly from the shaft in the deployed configuration. This may be achieved by each of the plurality of ultrasound transducer arrays being coupled to the shaft by a hinge. Pivoting of the hinge may thus enable configuring of the catheter.

It is noted that alternative means of deploying the ablation catheter may or may not involve changing an angle between each of the ultrasound transducer arrays and the shaft. A separation between the shaft and each of the arrays may in any case be increased in the deployed configuration relative to the folded configuration.

In this regard, the ablation catheter may comprise an expandable basket on which the plurality of ultrasound transducers arrays is mounted. In such an embodiment, the deployed configuration may be adopted upon expansion of the basket.

The ablation catheter may comprise at least three ultrasound transducer arrays. Providing at least three ultrasound transducer arrays may assist to ensure that at least of one of the ultrasound transducer arrays may be positioned so as to provide imaging of the target tissue, thereby to assist guiding of the ablation element and following of the ablative treatment.

The ultrasound transducer arrays may be evenly spaced relative to each other around the shaft in the deployed configuration. Even spacing of the ultrasound transducer arrays relative to each other may also assist to ensure that, upon deployment of the ablation catheter, at least one of the arrays is positioned to provide imaging of the target tissue. Uneven spacing of the ultrasound transducer arrays relative to each other is also conceivable.

The ablation catheter may comprise a balloon positioned between the plurality of ultrasound transducer arrays and the shaft, the deployed configuration being adopted upon inflation of the balloon. When the balloon is deflated, the ultrasound transducer arrays are against, or close to, the shaft such that the ablation catheter adopts the folded configuration.

The balloon may comprise a bulbous shape when inflated which extends from the shaft outwardly towards outer peripheries of the ultrasound transducer arrays. This shape may assist to minimize any trauma to tissue upon inflation of the balloon. The bulbous shape may, for instance, extend beyond the outer peripheries of the ultrasound transducer arrays. The balloon may thus assist to protect anatomical structures, such as chordae tendineae, from being damaged by the ultrasound transducer arrays when the ablation catheter is in the deployed configuration, or is in the process of adopting the deployed configuration.

The balloon may comprise a compliant material for conforming to anatomical features when the balloon is inflated. The compliant nature of the balloon may further avoid excessive forcing of the deployed ultrasound transducer arrays against the myocardium. Accordingly, unnecessary damage or excessive discomfort to the patient may be avoided.

The ablation catheter may comprise a plurality of sensing electrodes for measuring electrograms mounted on the balloon, the sensing electrodes being spatially separated relative to each other when the balloon is inflated. Alternatively, a plurality of such sensing electrodes for measuring electrograms may be mounted on the basket, i.e. when such a basket is included in the ablation catheter. The sensing electrodes may be spatially separated relative to each other when the basket is expanded.

As well as providing electrogram data which may assist in guiding or following the ablative treatment, the spatially distributed electrograms may also be used to determine which part of the balloon or basket is in contact with the tissue, since the amplitude of the detected signals will be weaker for minimal or no contact with cardiac tissue. This information may be used, for instance, in selecting which of the ultrasound transducer arrays to use for following the ablative treatment.

The ablation element may comprise a tip electrode for applying radiofrequency energy to tissue, the tip electrode being mounted on an end of the shaft. Radiofrequency ablation may be particularly appropriate for treating ventricular tachycardia. Other energy sources to ablate the myocardium such as cryocooling or laser heating may be used as well.

The ablation catheter may comprise at least one location sensor for tracking the position of the ablation element. Such location sensors may assist the clinician to guide the ablation element to the appropriate target tissue for correcting the arrhythmia.

The ablation catheter may comprise at least one aperture for supplying cooling fluid to an area being subject to the ablative treatment. Use of cooling fluid may assist to prevent unwanted excessive tissue damage, such as tissue charring, and also may assist to avoid blood coagulation, e.g. soft thrombus formation, during ablation.

According to another aspect of the present invention there is provided a catheter arrangement comprising: an ablation catheter according to any of the embodiments described above; and a guide catheter having a bore dimensioned to receive the ablation catheter in the folded configuration.

The guide catheter may, for example, be delivered to a position close to the target tissue. The ablation catheter in the folded configuration may then be fed through the bore of the guide catheter such that the ablation element reaches the target tissue. The ablation catheter may, for example, be deployed upon emergence of the ultrasound transducer arrays from an end of the guide catheter.

According to a further aspect of the present invention there is provided a system for providing ablative treatment of ventricular tachycardia comprising: an ablation catheter or a catheter arrangement as described above; and a display for displaying image data received by the plurality of ultrasound transducer arrays. The clinician may, for instance, use the displayed image data to control the ablation treatment such that the ablation treatment may be carried out to the requisite extent and terminated when sufficient tissue coagulation is deemed to have occurred.

### BRIEF DESCRIPTION OF THE DRAWINGS

Embodiments of the invention are described in more detail and by way of non-limiting examples with reference to the accompanying drawings, wherein:
FIG. 1 shows a cross-section of a portion of an ablation catheter in a deployed configuration according to an embodiment;
FIG. 2 shows another cross-section of the deployed ablation catheter shown in FIG. 1;
FIG. 3 shows a cross-section of the ablation catheter shown in FIG. 1 in a folded configuration;
FIG. 4 shows an orientation of the deployed ablation catheter shown in FIG. 1 with respect to a tissue surface;
FIG. 5 shows a further orientation of the deployed ablation catheter shown in FIG. 1 with respect to the tissue surface;
FIG. 6 schematically depicts the imaging planes provided by the ultrasound transducer arrays for the orientation shown in FIG. 4;
FIG. 7 schematically depicts the imaging planes provided by the ultrasound transducer arrays for the further orientation shown in FIG. 5;
FIG. 8 schematically depicts ablation of scar tissue for the further orientation shown in FIG. 5;
FIG. 9 shows a cross-section of a catheter arrangement according to an embodiment in which the ablation catheter is deployed;
FIG. 10 shows a cross-section of a catheter arrangement according to an embodiment in which the ablation catheter is partially withdrawn into the guide catheter; and
FIG. 11 shows an ultrasound imaging arrangement for general explanation of ultrasound imaging.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Provided is an ablation catheter for ablative treatment of ventricular tachycardia. The catheter comprises a shaft which is capable of being guided to the tissue to be ablated. An ablation element for ablating tissue is mounted on the shaft. The catheter further comprises a plurality of ultrasound transducer arrays for obtaining images of myocardial tissue. The arrays are positioned separately from each other around the circumference of the shaft. The catheter can adopt a folded configuration and a deployed configuration. In the folded configuration, the arrays are positioned against or close to the shaft thereby facilitating insertion and guiding of the catheter. In the deployed configuration, the arrays are more separated from the shaft than in the folded configuration.

The separation of the ultrasound transducer arrays from the shaft in the deployed configuration may result in at least one of the ultrasound transducer arrays projecting towards the target tissue, i.e. the tissue to be ablated. Projection of an ultrasound transducer array towards, e.g. against, the target tissue in this manner may facilitate assessment of the position of a scar lesion, e.g. from an infarction, or replacement fibrosis. Ultrasound imaging using the deployed ultrasound transducer arrays may thus enable effective guiding of the ablation treatment delivered via the ablation element to the target tissue.

Furthermore, such projection of at least one of the deployed ultrasound transducer arrays to the target tissue may assist in monitoring the ablation process. Thus it may be determined during course of the ablative treatment whether or not, for instance, tissue located between the ablation element and scar tissue located deeper (e.g. 1 cm depth) into the myocardium has been suitably ablated, i.e. coagulated. The capability to follow the ablation process using the deployed ultrasound transducer arrays may therefore assist to ensure that the ablation treatment is carried out to the requisite extent, and is terminated when sufficient tissue coagulation is deemed to have occurred.

The present invention may thus enhance the effectiveness of ablative treatment of ventricular tachycardia (VT), e.g. ischemic VT, ablation treatment. Moreover, the present invention may be applied to enhance ablative treatment of other cardiac arrhythmia conditions, such as atrial fibrillation.

Insertion of the ablation catheter into a patient in the deployed configuration risks causing unintended harm to the patient and damage to the ultrasound transducer arrays. For this reason, the ablation catheter is capable of adopting a folded configuration in which the arrays extend along the shaft of the catheter, i.e. against or close to the shaft, such that the form factor of the ablation catheter in the folded configuration is smaller than in the deployed configuration. The folded configuration thus ensures that the ablation catheter can be safely inserted into the patient, e.g. inside a suitable guide catheter, in a safe manner, and without undue risk of damage to the ultrasound transducer arrays during the process of insertion and guiding to the target tissue. Upon reaching the target tissue, the ablation catheter may be deployed, i.e. unfolded, thereby projecting one or more ultrasound transducer arrays towards, e.g. against, the target tissue, as previously described.

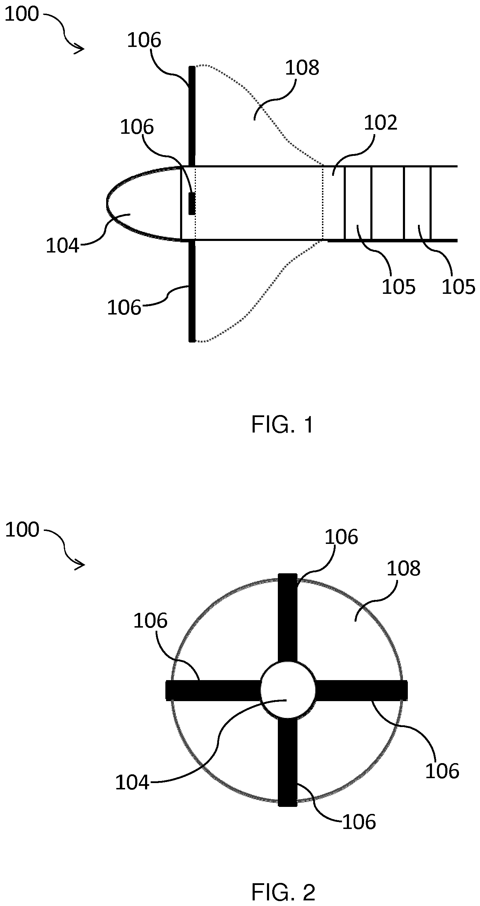

FIG. 1 shows a cross-section of a portion of an ablation catheter 100 according to an embodiment. The portion shown in FIG. 1 may be termed a 'distal portion' of the ablation catheter 100 given that during ablative treatment this portion is located inside a patient, e.g. in contact with myocardial tissue, and thus lies distal with respect to the clinician controlling the ablation catheter 100.

The ablation catheter 100 comprises a shaft 102 which permits the ablation catheter 100 to be guided to the target tissue. The shaft 102 is thus steerable in that the shaft 102 may be steered by the clinician, e.g. using a suitable steering mechanism, such that the distal portion reaches the target tissue. The ablation catheter 100 may accordingly be inserted into the vascular system and the distal portion may be passed into an atrium or ventricle of the heart, as is well-known per se.

The shaft 102 may comprise any suitable material, such as silicone rubber, nylon, polyurethane, polyethylene terephthalate (PET), latex, and thermoplastic elastomers. The shaft 102 may have a diameter which is dictated by the application of the ablation catheter 100. For example, the shaft 102 may be 5 Fr to 8 Fr (1.667 mm to 2.667 mm in diameter), such as 7 Fr (2.333 mm in diameter).

The ablation catheter 100 comprises an ablation element, which in this example is an electrode 104 mounted on the shaft 102. The ablation electrode 104 is suitable for ablating tissue which it contacts. In an embodiment, the ablation electrode 104 comprises a tip electrode for applying radiofrequency (RF) energy to the target tissue. As shown in FIG. 1, the tip electrode 104 may be mounted on an end of the shaft 102.

In such an embodiment, the RF energy may be applied to the target tissue located between the tip electrode 104 and a suitable grounding plate (not shown) which may be located on the surface of the patient's body. As is well-known per se for RF ablation, heat generation occurs within the target tissue, rather than within the ablation catheter 100. The size of the resulting lesion depends inter alia on the power delivered by the ablation electrode 104 and the ablation time. RF energy is applied via the tip electrode 104 to heat and coagulate the tissue that is in contact with the tip electrode 104 to a certain depth. By, for example, controlling the ablation time on the basis of ultrasound image data, the ablation treatment may be carried out to the requisite extent and terminated when sufficient tissue coagulation is deemed to have occurred.

Other electrode types, such as DC ablation electrodes, may alternatively or additionally be contemplated.

The ablation may instead be based on ablation techniques other than radiofrequency ablation, such as cryocooling or laser heating. Thus, more generally, there is an ablation element, which may be an electrode, or a device for coupling laser energy into the tissue, or a cryocooling tip.

The ablation electrode 104 may be fixed to the shaft 102 by any suitable method, such as by using a biocompatible adhesive.

As shown in FIG. 1, the ablation catheter 100 may comprise additional electrodes 105 which may extend around the circumference of the distal portion.

An ablation catheter typically has at least one additional electrode 105 adjacent to the ablation electrode 104 to enable the measurement of a local bipolar signal by subtracting the far field signals that is present on both signals. Apart from being the ablation electrode, the electrode 104 is thus also used to record the local tissue electrical activation. Typically, an additional pair of electrodes 105 is present in order to determine the bipolar signal between these electrodes. By comparing the shape and strengths of the different bipolar signals the physician can determine whether the different electrodes are close or further away from the myocardium. The electrodes 105 are thus a known feature of ablation catheters and they are not essential to the operation of the invention.

In an embodiment, the ablation catheter 100 is equipped with location sensors for tracking the position of the ablation electrode 104. Such location sensors may assist the clinician to guide the ablation electrode 104 to the appropriate tissue for correcting the arrhythmia.

The location sensors are used for 3D localization, to assist in navigation of the catheters in the human body, as is well known. Electromagnetic (EM) localization is a commonly used technique for this. It is based on a small coil in the tip of the catheter that senses the magnetic field strengths of a plurality of magnetic transmitters that are positioned close to the patient. The 3D position of the small coil with respect to these transmitters can be calculated by triangulation.

Another technology is known as Fiber Optic Real Shape which is used to determine the 3D shape and position of an optical fiber. This technology is also known as Optical Shape Sensing. This approach is for example disclosed in US 2011/0109898 and WO 2013/036247. When such a fiber is incorporated in a catheter, the 3D shape and position of the catheter can thus be determined.

Another approach is the use of a small ultrasound sensor at the tip of a catheter. By correlating the timing of the signal from the sensor that is present in an ultrasound field of an ultrasound imaging probe with the transmission scan times of the ultrasound beams, the position of the sensor in the ultrasound beams can be accurately determined and shown in the ultrasound image. The ultrasound transducers 106 could possibly be used as such a sensor in an externally applied ultrasound imaging field e.g. from TEE (transesophageal echo) or ICE (intracardiac echo) that may be used to visualize the position of the ablation catheter. This approach is for example disclosed in WO 2011/138698 and in the publication "A New Sensor Technology for 2D Ultrasound-Guided Needle Tracking", Huanxiang Lu et al, MICCAI 2014, Part II, LNCS 8674, pp. 389-10 396, 2014.

These are simply some known examples of catheter localization which may be used to guide the catheter, but they are not essential to the invention.

In an embodiment, the ablation catheter 100 comprises at least one aperture (not shown) for supplying cooling fluid to an area being subject to the ablative treatment. Use of cooling fluid may assist to prevent unwanted excessive tissue damage, such as tissue charring, and also may assist to avoid blood coagulation, e.g. soft thrombus formation, during ablation. The at least one aperture may, for instance, be located in the distal portion of the ablation catheter 100 and the cooling fluid, e.g. saline, may be supplied to the distal portion via a suitable flexible tube which may, for instance, be contained in the internal bore of the shaft 102.

The ablation catheter 100 shown in FIG. 1 is in a deployed configuration in which the ultrasound transducer arrays 106 radially extend outwardly from the shaft 102. In the embodiment shown in FIG. 1, the ablation catheter 100 comprises four ultrasound transducer arrays 106, of which three are visible in this cross-section: two arrays 106 in the plane of the figure, and one array 106 extending out of the plane of the figure towards the reader. In FIG. 2 which shows a front view of the ablation catheter 100 looking directly at the tip of the ablation electrode 104, the four ultrasound transducer arrays 106 are visible.

As shown in FIGs. 1 and 2, the ultrasound transducer arrays 106 are spatially separated around the circumference of the shaft 102 in the deployed configuration. In this manner, at least one of the spatially separated ultrasound transducer arrays 106 may extend towards the target tissue, e.g. myocardial tissue, in the deployed configuration. Accordingly, guiding of the ablation treatment delivered via the ablation electrode 104 to the target tissue and monitoring of the ablation process may be facilitated, as previously described.

Electrical wiring, coaxial cabling etc. (not shown) for connecting the ablation electrode 104 and ultrasound transducer arrays 106 to suitable control equipment may, for instance, be carried in the internal bore of the shaft 102, or alongside the shaft 102 when the ablation catheter is delivered to the target tissue using a suitable guide catheter (not shown in FIGs. 1-8, 11).

In non-limiting examples, each of the ultrasound transducer arrays 106 may comprise a linear phased array which generates a 2D ultrasound image slice extending normal to an emitting surface of the array. The linear array 106 may be elongated and have a long axis extending across its length. The plane of the 2D ultrasound image slice includes the long axis of the respective array 106. Ultrasound image slices may thus be generated around the periphery of the ablation electrode 104 which can, for instance, be used to determine the position of scar tissue from an infarction or replacement fibrosis.

Alternatively, the ultrasound transducer arrays 106 may generate 3D images. Simultaneous imaging by all ultrasound transducers may not be possible as the ultrasound echoes of one transducer may interfere with the imaging of the others. This may be addressed by using multiplexing techniques such that the transducers will image sequentially in rapid succession. Alternatively, the user may choose which transducer is employed for imaging.

In other non-limiting examples, an algorithm may be implemented to choose which of the ultrasound transducer arrays 106 will be used to provide image data based on an initial analysis how the respective arrays 106 are positioned with respect to the myocardium, the endocardial surface, the ablation electrode 104 etc.

The ultrasound transducer arrays 106 may comprise any suitable ultrasound transducer element, such as a piezoelectric micromachined ultrasound transducer (PMUT) or capacitive micromachined ultrasound transducer (CMUT).

The depth of the ultrasound imaging may, for example, be adjusted by tuning of the imaging frequency of the ultrasound transducer arrays 106. In this way, a more accurate determination of the required ablation lesion depth may be obtained.

The transducers may be tunable to generate an ultrasound frequency in a range of, for instance, 1 to 60 MHz, such as 10 to 40 MHz. At higher frequencies, e.g. 30 MHz, near field or close up imaging within a few mm or less from the ablation electrode 104 may be enabled. Lower frequencies, e.g. 15 MHz, may enable assessment of scar tissue located deeper into the myocardium.

Such frequency tuning may, for instance, be achieved by employing CMUT transducers in collapsed mode. In collapsed mode, the tension of the collapsed membranes is controlled by a bias voltage which determines the ultrasound frequency. Thus varying the bias voltage permits tuning of the frequency range of the transducers.

Ultrasound imaging using the ultrasound transducer arrays 106 may, for example, also be used to distinguish ablated tissue from viable myocardial tissue in order to enable monitoring of the ablation or coagulation process during RF energy application. Such techniques may, for instance, be based on measuring local cardiac contraction reduction resulting from ablation by strain or strain rate imaging. Alternatively, changes to local mechanical tissue properties, e.g. stiffness, when the tissue is subjected to ablation may be measured using shear wave elastography imaging, or by following changes in the structure of tissue using tissue characterization techniques based on features such as spectral parameters of ultrasound RF lines. Such techniques are well-known per se and will not be further described herein for the sake of brevity only.

In an embodiment, the ultrasound transducer arrays 106 may be evenly spaced relative to each other around the shaft 102 in the deployed configuration. Such even spacing of the ultrasound transducer arrays 106 may assist to ensure that at least of one of the ultrasound transducer arrays 106 may be positioned to provide imaging of the target tissue. In the embodiment shown in FIGs. 1 and 2, the four ultrasound transducer arrays 106 are positioned at regular intervals around the circumference of the shaft 102 such that a 90° angle separates neighboring ultrasound transducer arrays 106, as shown in FIG. 2.

Whilst the invention is illustrated in the Figures with an ablation catheter 100 having four spatially separated ultrasound transducer arrays 106, this is not intended to be limiting. In another embodiment, the ablation catheter 100 comprises at least two ultrasound transducer arrays 106. In another embodiment, the ablation catheter 100 comprises at least three ultrasound transducer arrays 106.

Alternatively, more than four ultrasound transducer arrays 106 may be included in the ablation catheter 100. For example, five, six, seven, eight, or more ultrasound transducer arrays 106 may be included in the ablation catheter 100. By providing greater numbers of ultrasound transducer arrays 106, the number of possible imaging planes correspondingly increases which may enhance the guiding and monitoring of the ablation treatment delivered by the ablation electrode 104. However, greater numbers of ultrasound transducer arrays 106 may increase the size, design complexity and cost of the ablation catheter 100, such that a balance must be struck in terms of the number of ultrasound transducer arrays 106 included in the ablation catheter 100.

In the embodiment shown in FIGs. 1 and 2, the ultrasound transducer arrays 106 are each coupled to the shaft 102 by a hinge (not visible). Pivoting of the hinge permits configuring of the ablation catheter 100, i.e. by unfolding of the ultrasound transducer arrays 106 from a folded configuration in which they are folded against, or close to, the shaft 102, to a deployed configuration in which the ultrasound transducer arrays 106 extend outwardly from the shaft 102.

In other words, in the folded configuration, the ultrasound transducer arrays 106 extend along, i.e. parallel with, a longitudinal axis of the shaft 102, such that the ablation catheter 100 may be inserted into a patient, e.g. using suitable guide catheter. The longitudinal axis of the shaft 102 may be defined as extending between the opposing ends of the shaft 102 when the shaft 102 adopts a linear conformation. In the deployed configuration, the ultrasound transducer arrays 106 may, for instance, extend radially outwards from this longitudinal axis, i.e. such that at least one of the ultrasound transducer arrays 106 may be projected towards, e.g. against, the target tissue.

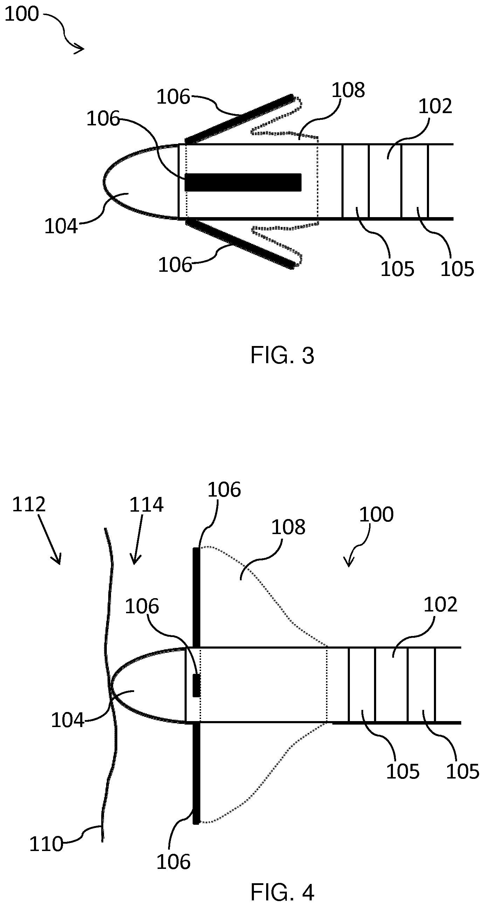

In an embodiment, the ablation catheter 100 comprises a balloon 108 positioned between the plurality of ultrasound transducer arrays 106 and the shaft 102. The deployed configuration of the ablation catheter 100 may be adopted by inflation of the balloon 108. When the balloon 108 is deflated, the ultrasound transducer arrays 106 are against, or close to, the shaft 102, as depicted in FIG. 3. Thus the folded ablation catheter 100 can be guided to the target tissue. Once the distal portion has been guided to the target tissue, i.e. is inside the requisite ventricle or atrium, the balloon 108 may be inflated. During inflation of the balloon 108, the ultrasound transducer arrays 106 may pivot with respect to the shaft 102 such that they extend outwardly from the shaft 102, as shown in FIG. 1.

Whilst the deployed configuration of the ablation catheter 100 as shown in FIG. 1 has the ultrasound transducer arrays 106 hinged at right angles to the shaft 102, this is not intended to be limiting. Depending, for instance, on the shape and degree of inflation of the balloon 108, the presence of anatomical features etc., the ultrasound transducer arrays 106 may be angled with respect to the shaft 102 by more or less than 90° in the deployed configuration.

The balloon 108 may comprise a compliant material for conforming to anatomical features when the balloon 108 is inflated. For example, the balloon 108 may comprise a suitable compliant polymer or rubber, such as cross-linked polyethylene, silicone, latex etc. The balloon 108 may be secured to the shaft 102 and/or to the ultrasound transducer arrays 106 by any suitable means such as using a biocompatible adhesive.

Inflation of the balloon 108 may be achieved by filling the balloon 108 with a suitable fluid, such as saline. In order to control the inflation of the balloon 108, the balloon 108 may, for example, be in fluid communication with a suitable syringe (not shown), e.g. a screw syringe. The saline may be supplied to the balloon 108 via a lumen running alongside or inside an internal bore of the ablation catheter 100.

The compliant nature of the balloon 108, and its inflated pressure, e.g. as controlled using the syringe, may permit the ultrasound transducer arrays 106 to be gently pushed towards and against the myocardium upon inflation of the balloon, as previously described. The compliant nature of the inflated balloon 108 may assist to avoid undue forcing of the ultrasound transducer arrays 106 against the myocardium. The ultrasound transducer arrays 106 may thus be permitted to push, e.g. pivot, back towards the shaft 102 in order to accommodate such anatomical structures. Unnecessary damage or excessive discomfort to the patient may thus be avoided.

In an embodiment, the balloon 108 comprises a bulbous shape when inflated. The bulbous shape may extend from the shaft 102 outwardly towards outer peripheries of the ultrasound transducer arrays 106. This shape may assist to minimize any trauma to tissue upon inflation of the balloon 108.

The balloon 108 may, for example, extend beyond these outer peripheries when inflated. In this manner, the balloon 108 may assist to protect anatomical structures, such as chordae tendineae, from being damaged by the ultrasound transducer arrays 106 when the ablation catheter 100 is in the deployed configuration, or is in the process of adopting the deployed configuration.

More generally, deployment of the ablation catheter 100 involves increasing the separation between the ultrasound transducer arrays 106 and the shaft 102 with respect to the folded configuration. This increased separation may permit at least one of the ultrasound transducer arrays 106 to be projected towards the target tissue, as previously described. Such deployment of the ablation catheter 100 may be achieved in any suitable way.

The plurality of ultrasound transducer arrays 106 may, for example, be directly mounted on the compliant balloon 108, i.e. without being directly coupled to the shaft 102. Inflation of the balloon 108 may thus cause the arrays 106 to become more separated from the shaft 102 in the deployed configuration.

In another embodiment, the ablation catheter 100 comprises an expandable basket (not shown) on which the plurality of ultrasound transducers arrays 106 is mounted. Such a basket may be formed, for instance, of flexible curved splines which bulge outwards from the shaft 102, e.g. upon emerging from a suitable guide catheter. By mounting the ultrasound transducer arrays 106 on these splines, the ultrasound transducer arrays 106 may be separated from the shaft 102 upon expansion of the basket, and thus one or more of the ultrasound transducer arrays 106 may be projected towards, e.g. against, the target tissue in the deployed configuration.

In embodiments wherein a balloon 108 or expandable basket is included to permit configuring of the ablation catheter 100, the balloon 108 or expandable basket may include a plurality of sensing electrodes (not shown) for measuring electrograms mounted on the balloon 108 or basket. The sensing electrodes may be spatially separated relative to each other upon inflation of the balloon 108 or, as the case may be, expansion of the basket.

The spatially distributed electrograms which may thus be measured using the plurality of sensing electrodes may also be used to determine which part of the balloon 108 or basket is in contact with the tissue, since the amplitude of the detected signals will be weaker for minimal or no contact with cardiac tissue. This information may be used, for instance, in selecting which of the ultrasound transducer arrays 106 to use for following the ablative treatment.

In a non-limiting example, the ultrasound transducer arrays 106 may also include an ablation electrode portion (not shown). In this manner, the ultrasound transducer arrays 106, as well as providing image data of the target tissue, may also contribute to ablation of the target tissue in the deployed configuration. The ablation electrode portion may include a conducting layer mounted on each of the plurality of transducer arrays 106. The conducting layer may be deposited on or adhered to each of the ultrasound transducer arrays 106 using any suitable technique, such as by vacuum deposition or using a biocompatible adhesive.

Alternatively or additionally, an ablation electrode portion may be mounted on the balloon 108 or basket, when the ablation catheter 100 comprises such a balloon 108 or basket.

FIG. 4 schematically depicts the deployed ablation catheter 100 with the ablation electrode 104 in contact with an endocardial surface 110. This surface 110 is the interface between the myocardium 112 and the blood 114 in the heart. In the scenario depicted in FIG. 4, the ablation electrode 104 is positioned against the myocardium 112 with the shaft 102 normal to the endocardial surface 110. All of the deployed ultrasound transducer arrays 106 face the tissue that is in contact with the ablation electrode 104.

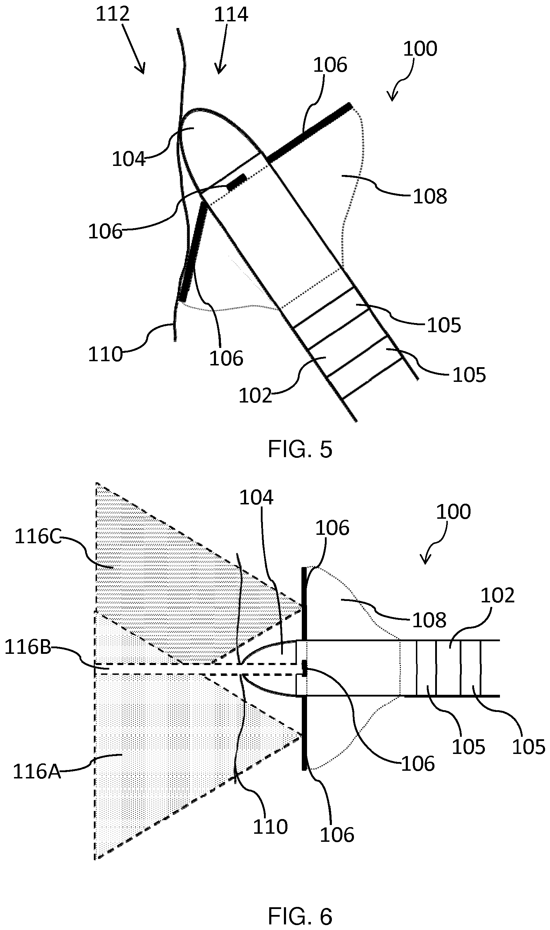

As shown in FIG. 5, when the angle of the ablation electrode 104 with respect to the endocardial surface 110 is changed relative to the normal orientation depicted in FIG. 4, the ultrasound transducer array or arrays 106 that are more proximal to the myocardium 112 is/are able to pivot to accommodate this angle and maintain gentle pressure on the tissue due, in part, to the compliant nature of the balloon 108. In other words, the compliant balloon 108 which serves to deploy the ultrasound transducer arrays 106, in combination with the hinges which couple the arrays 106 to the shaft 102 in this example, ensures that at least one of the ultrasound transducer arrays 106 is projected towards, i.e. faces, the tissue which is in contact with the ablation electrode 104, as previously described. It is noted that the orientation shown in FIG. 5 will be more common in practice than that shown in FIG. 4.

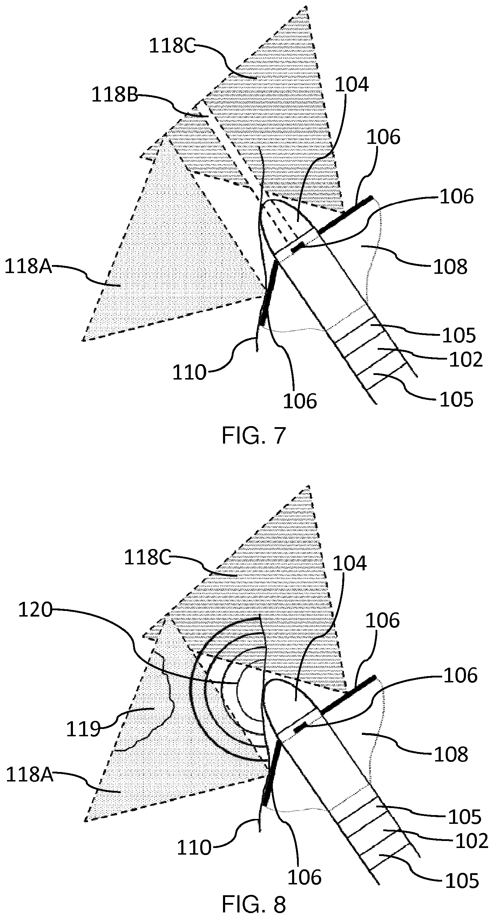

FIGs. 6 and 7 schematically depict the 2D image planes provided by the ultrasound transducer arrays 106 for the respective orientations of the ablation catheter 100 (relative to the endocardial surface 110) shown in FIGs. 4 and 5.

The ultrasound transducer arrays 106 may each be elongated and have a long axis extending across the length of the array 106. Each array 106 may generate a 2D image slice in a plane which includes this long axis. As shown in FIG. 6, in the case that the ablation catheter 100 is orientated normal to the tissue, the respective 2D image slices 116A, 116B and 116C of each of the transducer arrays 106 may overlap in the tissue area opposing the ablation electrode 104.

As shown in FIG. 7, when the ablation electrode 104 approaches the endocardial surface 110 at an angle, respective 2D image slices 118A, 118B and 118C are attained. At least one of the ultrasound transducer arrays 106 may image the tissue area beneath the ablation electrode 104, i.e. the array 106 which provides 2D image slice 118A in this example.

Projection of at least one of the ultrasound transducer arrays 106 towards (and against) the target area may facilitate assessment of the position of a scar lesion 119, e.g. from an infarction or replacement fibrosis, as shown in FIG. 8. Assessment of the position of the scar lesion 119 using the deployed ultrasound transducer arrays 106 may enable effective guiding of the ablation treatment delivered via the ablation electrode 104 to the target tissue.

The progress of the ablation front 120 (indicated by the concentric semicircles) may also be monitored using at least one of the deployed ultrasound transducer arrays 106 which is projected towards, e.g. against, the target tissue. The other ultrasound transducer arrays 106, i.e. which are not projected towards the target tissue, may provide additional imaging depending on their position with respect to the ablation electrode 104 and the endocardial surface 110.

The monitoring process may, for instance, involve using the ultrasound transducer array 106 which is most appropriately orientated with respect to the endocardial surface 110 to image the myocardium 112 and determine the position of the scar lesion 119. An initial scan may, for example, be carried out at a lower ultrasound frequency (e.g. 15 MHz) so as detect a scar lesion 119 lying deeper (e.g. 1 cm) into the myocardium 112, as previously described.

An appropriate imaging protocol may then be implemented to distinguish between ablated tissue, e.g. coagulated tissue, and viable myocardial tissue 112, e.g. using strain or strain rate imaging, shear wave elastography imaging, spectral parameters of ultrasound RF lines etc., as previously described.

RF energy may be applied using the ablation electrode 104, and the progress of the ablation front 120 may be monitored by the ultrasound transducer array(s) 106. During this monitoring process, higher ultrasound frequencies (e.g. 30 MHz) may, for instance, enable tissue visualization close to the ablation electrode 104, and lower frequencies (e.g. 15 MHz) may be employed to track the ablation to deeper tissue levels, e.g. where the scar lesion 119 may reside. Tuning of the ultrasound frequency provided by the ultrasound transducer arrays 106 may thus enable the clinician to judge that an adequate ablation depth has been reached. Thus it may be determined during the course of the ablative treatment whether or not, for instance, tissue located between the ablation electrode 104 and scar tissue 119 located deeper (e.g. 1 cm depth) into the myocardium 112 has been suitably ablated, i.e. coagulated.

Monitoring of the ablation depth in this manner may also provide a means to, for example, titrate the required RF energy and, in some cases, the supply of cooling fluid to the target tissue, i.e. supplied via the at least one aperture, to optimize ablation parameters. In this manner, the required depth of ablation may be reached without, for instance, excessive tissue damage being caused to tissue in immediate contact with the ablation electrode 104.

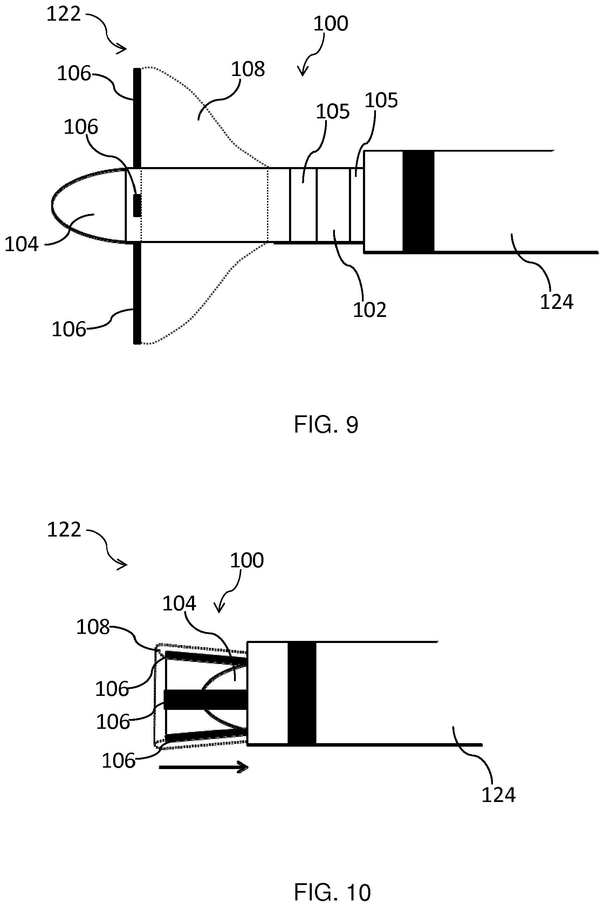

FIG. 9 shows a catheter arrangement 122 according to an embodiment. The catheter arrangement 122 includes an ablation catheter 100 as described above, and a guide catheter 124 having a bore dimensioned to receive the ablation catheter 100 in the folded configuration. The guide catheter 124 may, for example, be delivered to a position close to the target tissue. The ablation catheter 100 in the folded configuration may then be fed through the bore of the guide catheter 124. Once the distal portion of the ablation catheter 100 has emerged from the guide catheter 124, the ablation catheter 100 may be configured such that it adopts the deployed configuration, as shown in FIG. 9, and the ablative treatment may be administered.

Following the ablative treatment, the ablation catheter 100 may, for example, be retracted through the guiding catheter 124 while, or after, deflating the balloon 108. The direction of retraction of the ablation catheter 100 is represented by the arrow in FIG. 10. As shown in FIG. 10, once the balloon 108 has been deflated, the ultrasound transducer arrays 106 may be caused by the end of the guide catheter 124 to be pivoted against the ablation electrode 104 when the ablation catheter 100 is withdrawn into the guide catheter 124. The ablation catheter 100 can thus safely be retrieved from the body. Alternative means of re-folding the ablation catheter 100 for the purpose of withdrawing it from the patient, using a guide catheter 124 or otherwise, will be immediately apparent to the skilled person. For instance, an expandable basket carrying the ultrasound transducer arrays 106 may be recompressed into a folded state as it is withdrawn into the guide catheter 124.

In an embodiment, the ablation catheter 100 or catheter arrangement 122 is included in a system for providing ablative treatment of ventricular tachycardia. The system comprises a display for displaying image data received by the (deployed) plurality of ultrasound transducer arrays 106. The clinician may use the displayed image data to control the ablation treatment, e.g. the ablation time, such that the ablation treatment may be carried out to the requisite extent and terminated when sufficient tissue coagulation is deemed to have occurred.

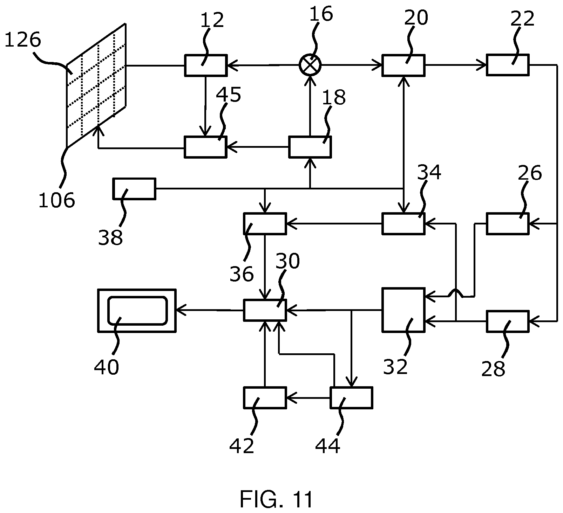

The general operation of an exemplary ultrasound imaging arrangement will now be described, with reference to FIG. 11. Such an imaging arrangement may, for instance, be included in the system for providing ablative treatment of ventricular tachycardia.

The arrangement comprises an ultrasound transducer array 106, e.g. a CMUT transducer array, for transmitting ultrasound waves and receiving echo information. The transducer array 106 may alternatively comprise piezoelectric transducers formed of materials such as PZT or PVDF. The transducer array 106 may be a two-dimensional array of transducers 126 capable of scanning in a 2D plane or in three dimensions for 3D imaging. In another example, the transducer array 106 may be a 1D array.

The transducer array 106 is coupled to a microbeamformer 12 in the probe which controls reception of signals by the CMUT array cells or piezoelectric elements. Microbeamformers are capable of at least partial beamforming of the signals received by subarrays (or "groups" or "patches") of transducers as described in US Patents 5,997,479 (Savord et al.), 6,013,032 (Savord), and 6,623,432 (Powers et al.).

Note that the microbeamformer is entirely optional. The examples below assume no analog beamforming.

The microbeamformer 12 is coupled by the probe cable to a transmit/receive (T/R) switch 16 which switches between transmission and reception and protects the main beamformer 20 from high energy transmit signals when a microbeamformer is not used and the transducer array 106 is operated directly by the main system beamformer. The transmission of ultrasound beams from the transducer array 106 is directed by a transducer controller 18 coupled to the microbeamformer by the T/R switch 16 and a main transmission beamformer (not shown), which receives input from the user's operation of the user interface or control panel 38.

One of the functions controlled by the transducer controller 18 is the direction in which beams are steered and focused. Beams may be steered straight ahead from (orthogonal to) the transducer array 106, or at different angles for a wider field of view. The transducer controller 18 can be coupled to control a DC bias control 45 for the CMUT array. The DC bias control 45 sets DC bias voltage(s) that are applied to the CMUT cells.

In the reception channel, partially beamformed signals are produced by the microbeamformer 12 and are coupled to a main receive beamformer 20 where the partially beamformed signals from individual patches of transducers are combined into a fully beamformed signal. For example, the main beamformer 20 may have 128 channels, each of which receives a partially beamformed signal from a patch of dozens or hundreds of CMUT transducer cells or piezoelectric elements. In this way the signals received by, in principle, thousands of transducers of a transducer array 106 can contribute efficiently to a single beamformed signal.

The beamformed reception signals are coupled to a signal processor 22. The signal processor 22 can process the received echo signals in various ways, such as band-pass filtering, decimation, I and Q component separation, and harmonic signal separation which acts to separate linear and nonlinear signals so as to enable the identification of nonlinear (higher harmonics of the fundamental frequency) echo signals returned from tissue and microbubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting the noise at higher frequencies from greater depths where these frequencies are devoid of anatomical information.

The beamformers for transmission and for reception are implemented in different hardware and can have different functions. Of course, the receiver beamformer is designed to take into account the characteristics of the transmission beamformer. In FIG. 11 only the receiver beamformers 12, 20 are shown, for simplicity. In the complete arrangement, there will also be a transmission chain with a transmission microbeamformer, and a main transmission beamformer.

The function of the microbeamformer 12 is to provide an initial combination of signals in order to decrease the number of analog signal paths. This is typically performed in the analog domain. The final beamforming is done in the main beamformer 20 and is typically after digitization.

The transmission and reception channels use the same transducer array 106 which has a fixed frequency band. However, the bandwidth that the transmission pulses occupy can vary depending on the transmission beamforming that has been used. The reception channel can capture the whole transducer bandwidth (which is the classic approach) or by using bandpass processing it can extract only the bandwidth that contains the useful information (e.g. the harmonics of the main harmonic).

The processed signals are coupled to a B mode (i.e. brightness mode, or 2D imaging mode) processor 26 and a Doppler processor 28. The B mode processor 26 employs detection of an amplitude of the received ultrasound signal for the imaging of structures in the body such as the tissue of organs and vessels in the body. B mode images of structure of the body may be formed in either the harmonic image mode or the fundamental image mode or a combination of both as described in US Pat. 6,283,919 (Roundhill et al.) and US Pat. 6,458,083 (Jago et al.) The Doppler processor 28 processes temporally distinct signals from tissue movement and blood flow for the detection of the motion of substances such as the flow of blood cells in the image field. The Doppler processor 28 typically includes a wall filter with parameters which may be set to pass and/or reject echoes returned from selected types of materials in the body.

The structural and motion signals produced by the B mode and Doppler processors are coupled to a scan converter 32 and a multi-planar reformatter 44. The scan converter 32 arranges the echo signals in the spatial relationship from which they were received in a desired image format. For instance, the scan converter may arrange the echo signal into a two dimensional (2D) sector-shaped format, or a pyramidal three dimensional (3D) image. The scan converter can overlay a B mode structural image with colors corresponding to motion at points in the image field with their Doppler-estimated velocities to produce a color Doppler image which depicts the motion of tissue and blood flow in the image field. The multi-planar reformatter will convert echoes which are received from points in a common plane in a volumetric region of the body into an ultrasound image of that plane, as described in US Pat. 6,443,896 (Detmer). A volume renderer 42 converts the echo signals of a 3D data set into a projected 3D image as viewed from a given reference point as described in US Pat. 6,530,885 (Entrekin et al.).

The 2D or 3D images are coupled from the scan converter 32, multi-planar reformatter 44, and volume renderer 42 to an image processor 30 for further enhancement, buffering and temporary storage for display on a display device 40. In addition to being used for imaging, the blood flow values produced by the Doppler processor 28 and tissue structure information produced by the B mode processor 26 are coupled to a quantification processor 34. The quantification processor 34 produces measures of different flow conditions such as the volume rate of blood flow as well as structural measurements such as the sizes of organs. The quantification processor 34 may receive input from the user control panel 38, such as the point in the anatomy of an image where a measurement is to be made. Output data from the quantification processor 34 is coupled to a graphics processor 36 for the reproduction of measurement graphics and values with the image on the display 40, and for audio output from the display device 40. The graphics processor 36 can also generate graphic overlays for display with the ultrasound images. These graphic overlays can contain standard identifying information such as patient name, date and time of the image, imaging parameters, and the like. For these purposes the graphics processor receives input from the user interface 38, such as patient name. The user interface 38 is also coupled to the transmit controller 18 to control the generation of ultrasound signals from the transducer array 106 and hence the images produced by the transducer array 106 and the ultrasound imaging arrangement. The transmit control function of the controller 18 is only one of the functions performed. The controller 18 also takes account of the mode of operation (given by the user) and the corresponding required transmitter configuration and band-pass configuration in the receiver analog to digital converter. The controller 18 can be a state machine with fixed states.

The user interface 38 is also coupled to the multi-planar reformatter 44 for selection and control of the planes of multiple multi-planar reformatted (MPR) images which may be used to perform quantified measures in the image field of the MPR images.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. An ablation catheter (100) for ablative treatment of ventricular tachycardia comprising:
a shaft (102);
an ablation element (104) mounted on said shaft;
a plurality of ultrasound transducer arrays (106) for obtaining images of myocardial tissue, the arrays being spaced relative to each other around the shaft, wherein the catheter is configurable to adopt a folded configuration in which the arrays extend along the shaft, and a deployed configuration in which the arrays are more separated from the shaft than in the folded configuration and **characterised in that** each of the plurality of ultrasound transducer arrays (106) is coupled to the shaft (102) by a hinge, pivoting of said hinge enabling configuring of said catheter.

2. The ablation catheter (100) of claim 1, wherein the ultrasound transducer arrays (106) extend outwardly from the shaft (102) in the deployed configuration.

3. The ablation catheter (100) of claim 1 or 2, comprising at least three ultrasound transducer arrays (106).

4. The ablation catheter (100) of any of claims 1-3, wherein the ultrasound transducer arrays (106) are evenly spaced relative to each other around the shaft (102) in the deployed configuration.

5. The ablation catheter (100) of any of claims 1-4, comprising a balloon (108) positioned between the plurality of ultrasound transducer arrays (106) and the shaft (102), said deployed configuration being adopted upon inflation of said balloon.

6. The ablation catheter (100) of claim 5, wherein the balloon (108) comprises a bulbous shape when inflated which extends from the shaft (102) outwardly towards outer peripheries of the ultrasound transducer arrays (106).

7. The ablation catheter (100) of claim 5 or 6, wherein the balloon (108) comprises a compliant material for conforming to anatomical features when said balloon is inflated.

8. The ablation catheter (100) of any of claims 5-7, comprising a plurality of sensing electrodes for measuring electrograms mounted on the balloon (108), said sensing electrodes being spatially separated relative to each other when the balloon is inflated.

9. The ablation catheter (100) of any of claims 1-3, comprising a guide catheter (124) having a bore dimensioned to receive the ablation catheter (100) in the folded configuration and an expandable basket on which the plurality of ultrasound transducers arrays (106) is mounted, said expandable basket comprising a plurality of flexible curved splines configured to bulge outwards from the shaft (102) when a distal portion of the ablation catheter (100) emerges from the guide catheter, said deployed configuration being adopted upon expansion of said basket, optionally wherein a plurality of sensing electrodes for measuring electrograms are mounted on the basket, said sensing electrodes being spatially separated relative to each other when the basket is expanded.

10. The ablation catheter (100) of any of claims 1-9, wherein the ablation element (104) comprises a tip electrode for applying radiofrequency energy to tissue, the tip electrode being mounted on an end of said shaft (102).

11. The ablation catheter (100) of any of claims 1-10, comprising at least one location sensor for tracking the position of said ablation element (104).

12. The ablation catheter (100) of any of claims 1-11, comprising at least one aperture for supplying cooling fluid to an area being subject to said ablative treatment.

13. A catheter arrangement (122) comprising:
an ablation catheter (100) of any of claims 1-12; and
a guide catheter (124) having a bore dimensioned to receive the ablation catheter in said folded configuration.

14. A system for providing ablative treatment of ventricular tachycardia comprising:
an ablation catheter (100) of any of claims 1-12 or a catheter arrangement (122) of claim 13; and
a display for displaying image data received by the plurality of ultrasound transducer arrays (106).

## Patentansprüche

1. Ablationskatheter (100) zur ablativen Behandlung von ventrikulärer Tachykardie, umfassend:
eine Welle (102);
ein Ablationselement (104), das auf der Welle angebracht ist;
eine Vielzahl von Ultraschallwandleranordnungen (106) zum Erhalten von Bildern von Myokardgewebe, wobei die Anordnungen relativ zueinander um den Schaft herum beabstandet sind, wobei der Katheter konfigurierbar ist, um eine gefaltete Konfiguration anzunehmen, bei der sich die Anordnungen entlang des Schafts erstrecken, und eine eingesetzte Konfiguration, bei der die Anordnungen stärker von der Welle getrennt sind als bei der gefalteten Konfiguration und **dadurch gekennzeichnet, dass** jede der mehreren Ultraschallwandleranordnungen (106) durch ein Scharnier mit der Welle (102) verbunden ist, wobei das Schwenken des Scharniers das Konfigurieren des Katheters ermöglicht.

2. Ablationskatheter (100) nach Anspruch 1, wobei sich die Ultraschallwandleranordnung (106) in der eingesetzten Konfiguration von der Welle (102) nach außen erstreckt.

3. Ablationskatheter (100) nach Anspruch 1 oder 2, umfassend mindestens drei Ultraschallwandleranordnungen (106).

4. Ablationskatheter (100) nach einem der Ansprüche 1 bis 3, wobei die Ultraschallwandleranordnungen (106) in der eingesetzten Konfiguration gleichmäßig relativ zueinander um den Schaft (102) herum beabstandet sind.

5. Ablationskatheter (100) nach einem der Ansprüche 1 bis 4, umfassend einen Ballon (108), der zwischen der Vielzahl von Ultraschallwandleranordnungen (106) und dem Schaft (102) positioniert ist, wobei die eingesetzte Konfiguration beim Aufblasen des Ballons übernommen wird.

6. Ablationskatheter (100) nach Anspruch 5, wobei der Ballon (108) beim Aufblasen eine bauchige Form aufweist, die sich vom Schaft (102) nach außen zu den Außenumfängen der Ultraschallwandleranordnungen (106) erstreckt.

7. Ablationskatheter (100) nach Anspruch 5 oder 6, wobei der Ballon (108) ein nachgiebiges Material zum Anpassen an anatomische Merkmale umfasst, wenn der Ballon aufgeblasen wird.

8. Ablationskatheter (100) nach einem der Ansprüche 5 bis 7, umfassend mehrere Erfassungselektroden zum Messen von Elektrogrammen, die an dem Ballon (108) angebracht sind, wobei die Erfassungselektroden räumlich relativ zueinander getrennt sind, wenn der Ballon aufgeblasen wird.

9. Ablationskatheter (100) nach einem der Ansprüche 1 bis 3, umfassend einen Führungskatheter (124) mit einer Bohrung, die so bemessen ist, dass sie den Ablationskatheter (100) in der gefalteten Konfiguration aufnimmt, und einem erweiterbaren Korb, auf dem die Vielzahl von Ultraschallwandleranordnungen (106) angebracht sind, wobei der erweiterbare Korb mehrere flexibel gekrümmte Rillen umfasst, die so konfiguriert sind, dass sie sich vom Schaft (102) nach außen wölben, wenn ein distaler Abschnitt des Ablationskatheters (100) aus dem Führungskatheter austritt, wobei die eingesetzte Konfiguration beim Ausdehnen des Korbs angenommen ist, wobei gegebenenfalls mehrere Erfassungselektroden zum Messen von Elektrogrammen an dem Korb angebracht sind, wobei die Erfassungselektroden beim Ausdehnen des Korbs räumlich relativ zueinander getrennt sind.

10. Ablationskatheter (100) nach einem der Ansprüche 1 bis 9, wobei das Ablationselement (104) eine Spitzenelektrode zum Anwenden von Hochfrequenzenergie auf Gewebe umfasst, wobei die Spitzenelektrode an einem Ende des Schafts (102) angebracht ist.

11. Ablationskatheter (100) nach einem der Ansprüche 1 bis 10, umfassend mindestens einen Positionssensor zum Verfolgen der Position des Ablationselements (104).

12. Ablationskatheter (100) nach einem der Ansprüche 1 bis 11, umfassend mindestens eine Öffnung zum Zuführen von Kühlflüssigkeit zu einem Bereich, der der ablativen Behandlung unterzogen wird.

13. Katheteranordnung (122), umfassend:
einen Ablationskatheter (100) nach einem der Ansprüche 1 bis 12; und
einen Führungskatheter (124) mit einer Bohrung, die bemessen ist, um den Ablationskatheter in der gefalteten Konfiguration aufzunehmen.

14. System zum Bereitstellen der ablativen Behandlung von ventrikulärer Tachykardie, umfassend:
einen Ablationskatheter (100) nach einem der Ansprüche 1 bis 12 oder eine Katheteranordnung (122) nach einem der Ansprüche 13; und
eine Anzeige zum Anzeigen von Bilddaten, die von mehreren Ultraschallwandleranordnungen (106) empfangen werden.

## Revendications

1. Cathéter d'ablation (100) pour le traitement ablatif de la tachycardie ventriculaire comprenant:
un arbre (102);
un élément d'ablation (104) monté sur ledit arbre;
une pluralité de réseaux de transducteurs ultrasonores (106) pour obtenir des images de tissu myocardique, les réseaux étant espacés les uns par rapport aux autres autour de l'arbre, où le cathéter est configurable pour adopter une configuration pliée dans laquelle les réseaux s'étendent le long de l'arbre, et une configuration déployée dans laquelle les réseaux sont plus séparés de l'arbre que dans la configuration pliée et **caractérisé en ce que** chacun de la pluralité de réseaux de transducteurs ultrasonores (106) est couplé à l'arbre (102) par une charnière, le pivotement de ladite charnière permettant la configuration de ledit cathéter.

2. Cathéter d'ablation (100) selon la revendication 1, dans lequel le réseau de transducteurs ultrasonores (106) s'étend vers l'extérieur à partir de l'arbre (102) dans la configuration déployée.

3. Cathéter d'ablation (100) selon la revendication 1 ou 2, comprenant au moins trois réseaux de transducteurs ultrasonores (106).

4. Cathéter d'ablation (100) selon l'une quelconque des revendications 1 à 3, dans lequel les réseaux de transducteurs ultrasonores (106) sont régulièrement espacés les uns par rapport aux autres autour de l'arbre (102) dans la configuration déployée.

5. Cathéter d'ablation (100) selon l'une quelconque des revendications 1 à 4, comprenant un ballonnet (108) positionné entre la pluralité de réseaux de transducteurs ultrasonores (106) et l'arbre (102), ladite configuration déployée étant adoptée lors du gonflage dudit ballonnet.

6. Cathéter d'ablation (100) selon la revendication 5, dans lequel le ballonnet (108) comprend une forme bulbeuse lorsqu'il est gonflé qui s'étend de l'arbre (102) vers l'extérieur vers les périphéries externes des réseaux de transducteurs ultrasonores (106).

7. Cathéter d'ablation (100) selon la revendication 5 ou 6, dans lequel le ballonnet (108) comprend un matériau souple pour se conformer aux caractéristiques anatomiques lorsque ledit ballonnet est gonflé.

8. Cathéter d'ablation (100) selon l'une quelconque des revendications 5 à 7, comprenant une pluralité d'électrodes de détection pour mesurer des électrogrammes montés sur le ballonnet (108), lesdites électrodes de détection étant spatialement séparées l'une par rapport à l'autre lorsque le ballonnet est gonflé.

9. Cathéter d'ablation (100) selon l'une quelconque des revendications 1 à 3, comprenant un cathéter de guidage (124) ayant un alésage dimensionné pour recevoir le cathéter d'ablation (100) dans la configuration pliée et un panier extensible sur lequel la pluralité de transducteurs ultrasonores des réseaux (106) est montée, ledit panier extensible comprenant une pluralité de cannelures courbes flexibles configurées pour se bomber vers l'extérieur à partir de l'arbre (102) lorsqu'une partie distale du cathéter d'ablation (100) émerge du cathéter de guidage, ladite configuration déployée étant adoptée lors de l'expansion dudit panier, optionnellement où une pluralité d'électrodes de détection pour mesurer des électrogrammes sont montées sur le panier, lesdites électrodes de détection étant spatialement séparées les unes par rapport aux autres lorsque le panier est déployé.

10. Cathéter d'ablation (100) selon l'une quelconque des revendications 1 à 9, dans lequel l'élément d'ablation (104) comprend une électrode à pointe pour appliquer une énergie radiofréquence au tissu, l'électrode à pointe étant montée sur une extrémité dudit arbre (102).

11. Cathéter d'ablation (100) selon l'une quelconque des revendications 1 à 10, comprenant au moins un capteur de localisation pour suivre la position dudit élément d'ablation (104).

12. Cathéter d'ablation (100) selon l'une quelconque des revendications 1 à 11, comprenant au moins une ouverture pour fournir un fluide de refroidissement à une zone étant soumise audit traitement ablatif.

13. Agencement de cathéter (122) comprenant:
un cathéter d'ablation (100) selon l'une quelconque des revendications 1 à 12; et
un cathéter de guidage (124) ayant un alésage dimensionné pour recevoir le cathéter d'ablation dans ladite configuration pliée.

14. Système pour fournir un traitement ablatif de la tachycardie ventriculaire comprenant:
un cathéter d'ablation (100) selon l'une quelconque des revendications 1 à 12 ou un agencement de cathéter (122) selon la revendication 13; et
un afficheur pour afficher des données d'image reçues par la pluralité de réseaux de transducteurs ultrasonores (106).
